# EUROPÄISCHE PATENTANMELDUNG

(11) **EP 0 916 695 A1**
(43) Veröffentlichungstag der Anmeldung: **19.05.1999**
(21) Anmeldenummer: 98811107.6
(22) Anmeldetag: 05.11.1998
(51) Int. Cl.: C08K 5/372, C08K 5/41, C09K 15/28, C07C 323/60, C07C 317/44

(54) **Thiodipropionsäure-bis-amide als Stabilisatoren für helle Elastomere**

(30) Priorität: 14.11.1997 CH 2641/97
(71) Anmelder: Ciba Specialty Chemicals Holding Inc., 4057 Basel (CH)
(72) Erfinder: Meier, Hans-Rudolf, 1723 Marly (CH); Knobloch, Gerrit, 4312 Magden (CH)

(57) **Zusammenfassung**

Helle Elastomere, welche eine hervorragende Stabilität gegenüber oxidativen, licht- oder ozoninduzierten Abbau besitzen, enthalten als Stabilisatoren mindestens eine Verbindung der Formel I 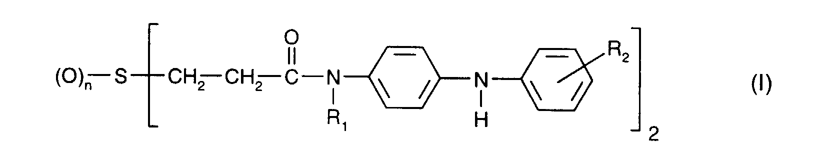 worin
R₁ Wasserstoff, C₁-C₁₂-Alkyl, Cyclohexyl, Phenyl oder Benzyl bedeutet,
R₂ Wasserstoff oder C₁-C₄-Alkyl darstellt, und
n die Zahl 0, 1 oder 2 bedeutet. Die Verbindungen der Formel I eignen sich hervorragend als farbstabile und nicht-verfärbende Stabilisatoren und Antiozonantien für helle Elastomere gegen oxidativen, thermischen, licht- oder ozoninduzierten Abbau.

## Beschreibung

Die vorliegende Erfindung betrifft Zusammensetzungen enthaltend ein dem oxidativen, thermischen, licht- oder ozoninduzierten Abbau unterworfenes helles Elastomer und als Stabilisator mindestens ein Thiodipropionsäure-bis-amid, sowie die Verwendung derselben als farbstabile und nicht-verfärbende Stabilisatoren und Antiozonantien für helle Elastomere gegen oxidativen, thermischen, licht- oder ozoninduzierten Abbau, sowie ein Verfahren zur Stabilisierung und zur Verminderung der Verfärbung von hellen Elastomeren, dadurch gekennzeichnet, dass man diesen mindestens ein Thiodipropionsäure-bis-amid einverleibt oder auf diese aufbringt.

Gummiartikel (Vulkanisate) unterliegen, wie alle Polymere, dem oxidativen, thermischen oder lichtinduzierten Abbau. Eine besonders schädigender Faktor von Dienkautschuk-Vulkanisaten ist Ozon. Ozon greift die im Gummi (Vulkanisat) noch zahlreich vorhandenen Kohlenstoff-Kohlenstoff-Doppelbindungen an und führt über den als Ozonolyse bekannten Mechanismus zu Schädigungen, die sich durch typische Oberflächenrissbildung äussert und zu einem Versagen des Gummiartikels führt. Besonders gravierend sind die Schädigungen bei dynamischer Beanspruchung des Gummiartikels.

Zur Verhinderung von Ozonschädigungen werden Vulkanisaten in der Regel Alterungsschutzmittel aus der Klasse der para-Phenylen-diamine zugesetzt [siehe Russel A. Mazzeo et al., "Tire Technology International" 1994, Seiten 36-46; oder Donald E. Miller et al., Rubber World, 200 (5), 13-23 (1989)]. Diese Verbindungen haben, vor allem unter dynamischen Bedingungen, eine sehr gute Schutzwirkung, entwickeln aber eine starke Eigenfarbe ("discoloring") und zeigen, bedingt durch die hohen Migrationsraten, eine intensive Kontaktverfärbung ("staining"), d.h. der Farbstoff geht bei direktem Kontakt auf andere Substrate/Artikel über. Die als Stand der Technik eingesetzten Stabilisatoren können daher nicht für russfreie oder "nichtschwarze" Gummiartikel verwendet werden und eignen sich auch nicht für russhaltige (schwarze) Gummiartikel, die bestimmungsgemäss im direkten Kontakt mit hellfarbigen Artikeln verwendet werden.

Es besteht daher weiterhin ein Bedarf an farbstabilen Stabilisatoren, die hellfarbige Gummiartikel gegen Ozon schützen. Ebenso besteht weiterhin ein Bedarf an Stabilisatoren, die zwar eine Eigenfarbe besitzen können, aber durch beispielsweise chemische Bindung an die Kautschukketten nicht in der Lage sind, die Farbe auf andere Artikel zu übertragen.

Es wurde nun gefunden, dass sich bestimmte Thiodipropionsäure-bis-amide sich besonders gut als Stabilisatoren für helle Elastomere, die gegen oxidativen, thermischen, licht- oder ozoninduzierten Abbau empfindlich sind, eignen.

Die vorliegende Erfindung betrifft daher Zusammensetzungen enthaltend
a) ein dem oxidativen, thermischen, licht- oder ozoninduzierten Abbau unterworfenes helles Elastomer, und
b) mindestens eine Verbindung der Formel I 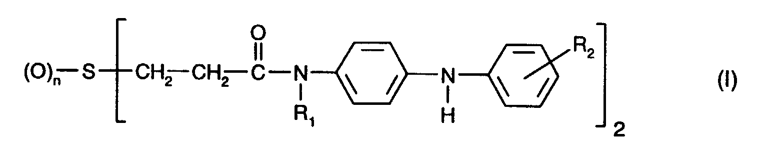 worin
R₁ Wasserstoff, C₁-C₁₂-Alkyl, Cyclohexyl, Phenyl oder Benzyl bedeutet,
R₂ Wasserstoff oder C₁-C₄-Alkyl darstellt, und
n die Zahl 0, 1 oder 2 bedeutet.

Alkyl mit bis zu 12 Kohlenstoffatomen bedeutet einen verzweigten oder unverzweigten Rest wie beispielsweise Methyl, Ethyl, Propyl, isopropyl, n-Butyl, sec-Butyl, isobutyl, tert-Butyl, 1,1-Dimethyl-1-propyl, 2-Ethylbutyl, n-Pentyl, isopentyl, 2-Pentyl, 2-Hexyl, 2-Heptyl, 2-Octyl, n-Octyl, Nonyl, Decyl, Undecyl oder Dodecyl. Eine bevorzugte Bedeutung von R₁ ist beispielsweise C₃-C₁₂-Alkyl, insbesondere C₃-C₈-Alkyl, z.B. Isopropyl. Eine besonders bevorzugte Bedeutung von R₂ ist beispielsweise Methyl und Ethyl.

Bevorzugt sind Zusammensetzungen enthaltend als Komponente (b) mindestens eine Verbindung der Formel I, worin
R₁ Wasserstoff oder C₃-C₈-Alkyl bedeutet,
R₂ Wasserstoff oder Methyl darstellt, und
n die Zahl 0 oder 1 bedeutet.

Besonders bevorzugt sind Zusammensetzungen enthaltend als Komponente (b) mindestens eine Verbindung der Formel I, worin
R₁ Wasserstoff oder C₃-C₈-Alkyl bedeutet,
R₂ Wasserstoff darstellt, und
n die Zahl 0 oder 1 bedeutet.

Die Verbindungen der Formel I sind in der Literatur teilweise bekannt und können beispielsweise ausgehend von den bekannten Verbindungen der Formel II 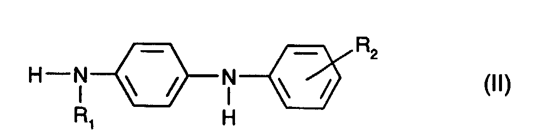 mit einem halben Equivalent Thio-3,3-bis-propionsäuredichlorid [Herstellung siehe H. Schmid et al., Helvetica Chimica Acta 34, 894-897 (1951)] zu den Verbindungen der Formel I, worin n = 0 ist, umgesetzt werden. Die Herstellung der Verbindungen der Formel I, worin n die Zahl 1 oder 2 bedeutet, erfolgt beispielsweise durch Oxidation der Verbindungen der Formel I, worin n = 0 ist, mit einer wässrigen Wasserstoffperoxid-Lösung.

Die Komponente (b) eignet sich zum Stabilisieren von hellen Elastomeren gegen oxidativen, thermischen, licht- oder ozoninduzierten Abbau.

Unter Elastomeren werden makromolekulare Werkstoffe verstanden, die bei Raumtemperatur nach einer erheblichen Verformung durch geringe Belastung schnell wieder annähernd ihre ursprüngliche Gestalt anzunehmen vermögen. Siehe auch Hans-Georg Elias, "An Introduction to Polymer Science", Kapitel 12. "Elastomers", Seiten 388-393, 1997, VCH Verlagsgesellschaft mbH, Weinheim, Deutschland; oder "Ullmann's Encyclopedia of Industrial Chemistry, Fifth, Completely Revised Edition, Volume A 23", Seiten 221-440 (1993).

Als Elastomere können die erfindungsgemässen Zusammensetzungen beispielsweise folgende Materialien enthalten:
1. Polymere von Diolefinen, wie beispielsweise Polybutadien oder Polyisopren.
2. Copolymere von Mono- und Diolefinen untereinander oder mit anderen Vinylmonomeren, wie z.B. Propylen-lsobutylen-Copolymere, Propylen-Butadien-Copolymere, Isobutylen-lsopren-Copolymere, Ethylen-Alkylacrylat-Copolymere, Ethylen-Alkylmethacrylat-Copolymere, Ethylen-Vinylacetat-Copolymere, Acrylnitril/Butadien-Copolymere sowie Terpolymere von Ethylen mit Propylen und einem Dien, wie Hexadien, Dicyclopentadien oder Ethylidennorbornen.
3. Copolymere von Styrol oder α-Methylstyrol mit Dienen oder Acrylderivaten, wie z.B. Styrol-Butadien, Styrol-Butadien-Alkylacrylat und -methacrylat; sowie Block-Copolymere des Styrols, wie z.B. Styrol-Butadien-Styrol oder Styrol-lsopren-Styrol.
4. Halogenhaltige Polymere, wie z.B. Polychloropren, Chlorkautschuk, chloriertes und bromiertes Copolymer aus Isobutylen-isopren (Halobutylkautschuk).
5. Naturkautschuk.
6. Wässrige Emulsionen natürlicher oder synthetischer Kautschuke, wie z.B. Naturkautschuk-Latex oder Latices von carboxylierten Styrol-Butadien-Copolymeren.

Bevorzugt handelt es sich bei den zu schützenden hellen Elastomeren um helle vulkanisierte Elastomere. Besonders bevorzugt sind helle (russfreie oder "nichtschwarze") Polydien-Vulkanisate oder helle halogenhaltige Polydien-Vulkanisate, insbesondere helle (russfreie oder "nichtschwarze") Styrol-Butadien-Copolymer-Vulkanisate.

Die Komponente (b) wird dem zu stabilisierenden Elastomer zweckmässig in einer Menge von 0,2 bis 10 %, beispielsweise 0,5 bis 5 %, vorzugsweise 0,8 bis 3,0 %, bezogen auf das Gewicht des zu stabilisierenden Elastomers, zugesetzt.

Zusätzlich zu der Komponenten (a) und (b) können die erfindungsgemässen Zusammensetzungen zusätzlich weitere Additive enthalten, wie beispielsweise die folgenden:

### 1. Antioxidantien

1.1. Alkylierte Monophenole, z.B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Di-nonyl-4-methylphenol, 2,4-Dimethyl-6-(1'-methyl-undec-1'-yl)-phenol, 2,4-Dimethyl-6-(1'-methylheptadec-1'-yl)-phenol, 2,4-Dimethyl-6-(1'-methyl-tridec-1'-yl)-phenol und Mischungen davon.
1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.
1.4. Tocopherole, z.B. α-Tocopherol, β-Tocopherol, γ-Tocopherol, δ-Tocopherol und Mischungen davon (Vitamin E).
1.5. Hydroxylierte Thiodiphenylether, z.B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis-(6-tert-butyl-2-methylphenol), 4,4'-Thio-bis(3,6-di-sec.-amylphenol), 4,4'-Bis(2,6-dimethyl-4-hydroxyphenyl)-disulfid.
1.6. Alkyliden-Bisphenole, z.B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(α-methylbenzyl)4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol-bis[3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis(3,5-di-tert-butyl-4-hydroxyphenyl)-propan, 2,2-Bis(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.
1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterephthalat, Bis(3,5-di-tert-butyl-4-hydroxybenzyl)sulfid, Isooctyl-3,5-di-tert-butyl-4-hydroxybenzyl-mercaptoacetat.
1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.
1.9. Hydroxybenzyl-Aromaten, z.B. 1,3,5-Tris(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris-(3,5-di-tert-butyl-4-hydroxybenzyl)-phenol.
1.10. Triazinverbindungen, z.B. 2,4-Bis-octylmercapto-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicydohexyl-4-hydroxybenzyl)-isocyanurat.
1.11. Benzylphosphonate, z.B. Dimethyl-2,5-di-tert-butyl-4-hydroxybenzylphosphonat, Di-ethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
1.13. Ester der β-(3,5-Di-tert-butyl-4-hydroxyphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2]-octan.
1.14. Ester der β-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(hydroxyethyl)-oxalsäurediamid, 3-Thlaundecanol, 3-Thiapentadecanol, Trimethylhexandiol, Trimethylolpropan, 4-Hydroxymethyl-1-phospha-2,6,7-trioxabicyclo-[2.2.2]-octan.
1.15. Ester der β-(3,5-Dicyclohexyl-4-hydroxyphenyl)-propansäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2)-octan.
1.17. Amide der β-(3,5-Di-tert-butyl-4-hydroxyphenyl)-propionsäure, wie z.B. N,N'-Bis(3,5-di-tert-butyl-4-hydroxyphenylpropionyl)-hexamethylendiamid, N,N'-Bis(3,5-di-tert-butyl-4-hydroxyphenylpropionyl)-trimethylendiamid, N,N'-Bis(3,5-di-tert-butyl-4-hydroxyphenylpropionyl)-hydrazid, N,N'-Bis[2-(3-[3,5-di-tert-butyl-4-hydroxyphenyl]-propionyloxy)ethyl]oxamid (Naugard®XL-1 der Firma Uniroyal).
1.18. Ascorbinsäure (Vitamin C).
1.19. Aminische Antioxidantien, wie z.B. N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(2-naphthyl)-p-phenylendiamin, N-lsopropyi-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Altyldiphenylamin, 4-lsopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylaminophenol, 4-Nonanoylamino-phenol, 4-Dodecanoylamino-phenol, 4-Octadecanoylaminophenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diaminodiphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/lsohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethypiperidin-4-on, 2,2,6,6-Tetramethylpiperidin-4-ol.

### 2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis(α,α-dimethylbenzyl)-2'-hydroxyphenyl)-benzotriazol, 2-(3'-tert-Butyl-2'-hydroxy-5'-(2-octyloxycarbonylethyl)phenyl)-5-chlor-benzotriazol, 2-(3'-tert-Butyl-5'-(2-(2-ethylhexyloxy)-carbonylethyl]-2'-hydroxyphenyl)-5-chlor-benzotriazol, 2-(3'-tert-Butyl-2'-hydroxy-5'-(2-me-thoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyi-2'-hydroxy-5'-(2-octyloxycarbonylethyl)-phenyl)-benzotriazol, 2-(3'-tert-Butyl-5'-[2-(2-ethylhexyloxy)carbonylethyi]-2'-hydroxyphenyl)-benzotriazol, 2-(3'Dodecyl-2'-hydroxy-5'-methylphenyl)-benzotriazol, 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'tert-Butyl-5'-(2-methoxycarbonylethyl)-2'-hydroxy-phenyl]-benzotriazol mit Polyethylenglycol 300; 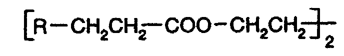 mit R = 3'-tert-Butyl-4'-hydroxy-5'-2H-benzotriazol-2-yl-phenyl; 2-[2'-Hydroxy-3'-(α,α-dimethylbenzyl)-5'-(1,1,3,3-tetramethylbutyl)-phenyl]-benzotriazol; 2-[2'-Hydroxy-3'-(1,1,3,3-tetramethylbutyl)-5'-(α,α-dimethylbenzyl)-phenyl]-benzotriazol.
2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw.-butylester, α-Carbomethoxy-p-methoxy-zimtsäure-methylester, N-(β-Carbomethoxy-β-cyanovinyl)-2-methyl-indolin.
2.5. Nickelverbindungen, wie z.B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
2.6. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
2.7. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propyloxy)phenyl]-4,6-bis(2,4-dimethylphenyl)-1,3,5-triazin, 2-[2-Hydroxy-4-(2-hydroxy-3-octyloxy-propyloxy)phenyl]-4,6-bis(2,4-dimethylphenyl)-1,3,5-triazin, 2-[4-(dodecyloxy/tridecyloxy-2-hydroxypropoxy)-2-hydroxy-phenyl]-4,6-bis(2,4-dimethylphenyl)-1,3,5-triazin, 2-[2-Hydroxy-4-(2-hydroxy-3-dodecyloxy-propoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin, 2-(2-Hydroxyphenyl)-4-(4-methoxyphenyl)-6-phenyl-1,3,5-triazin, 2-{2-Hydroxy-4-[3-(2-ethylhexyl-1-oxy)-2-hydroxypropyloxy]phenyl)-4,6-bis(2,4-dimethylphenyl)-1,3,5-triazin.
3. Metalldesaktivatoren, wie z.B. N,N'-Diphenyloxalsäurediamid, N-Salicylal-N'-salicyloylhydrazin, N,N'-Bis(salicyloyl)-hydrazin, N,N'-Bis(3,5-di-tert-butyl-4-hydroxyphenylpropionyl)-hydrazin, 3-Salicyloylamino-1,2,4-triazol, Bis(benzyliden)-oxalsäuredihydrazid, Oxanilid, lsophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Phosphite und Phosphonite, wie z.B. Triphenylphosphit, Diphenylalkylphosphite, Phenyldialkylphosphite, Tris(nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-lsooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis(2,4-di-tert-butyl-6-methylphenyl)-methylphosphit, Bis(2,4-di-tert-butyl-6-methylphenyl)-ethylphosphit, 2,2',2"-Nitrilo[triethyl-tris(3,3',5,5'-tetra-tert-butyl-1,1'-biphenyl-2,2'-diyl)-phosphit], 2-Ethylhexyl-(3,3',5,5'-tetra-tert-butyl-1,1'-biphenyl-2,2'-diyl)-phosphit.
5. Hydroxylamine wie z.B. N,N-Dibenzylhydroxylamin, N,N-diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylamin aus hydrierten Talgfettaminen.
6. Nitrone wie z.B. N-Benzyl-alpha-phenyl-nitron, N-Ethyl-alpha-methyl-nitron, N-Octyl-alpha-heptyl-nitron, N-Lauryl-alpha-undecyl-nitron, N-Tetradecyl-alpha-tridecyl-nitron, N-Hexadecyl-alpha-pentadecyl-nitron, N-Octadecyl-alpha-heptadecyl-nitron, N-Hexadecyl-alpha-heptadecyl-nitron, N-Octadecyl-alpha-pentadecyl-nitron, N-Heptadecyl-alpha-heptadecyl-nitron, N-Octadecyl-alpha-hexadecyl-nitron, Nitrone abgeleitet von N,N-Dialkylhydroxylaminen hergestellt aus hydrierten Talgfettaminen.
7. Thiosyneroisten wie z.B. Thiodipropionsäure-di-laurylester oder Thiodipropionsäure-distearylester.
8. Peroxidzerstörende Verbindungen, wie z.B. Ester der B-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis(β-dodecylmercapto)-propionat.
9. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinkbrenzcatechinat.
10. Nukleierunosmittel, wie z.B. anorganische Stoffe wie z.B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z.B. 4-tert-Butylbenzoesäure, Adipinsäure, Diphenylessigsäure, Natriumsuccinat oder Natriumbenzoat; polymere Verbindungen wie z.B. ionische Copolymerisate ("Ionomere").
11. Füllstoffe und Verstärkungsmittel, wie z.B. Russ, Calciumcarbonat, Silikate, Glasfasern, Glaskugeln, Talk, Kaolin, Glimmer, Bariumsulfat, Metalloxide und -hydroxide, Graphit, Holzmehl und Mehle oder Fasern anderer Naturprodukte, synthetische Fasern.
12. Sonstige Zusätze, wie z.B. Weichmacher wie beispielsweise Mineralöle oder Dioctylphthalat, Gleitmittel, Emulgatoren, Pigmente wie beispielsweise Titandioxid, Rheologieadditive, Katalysatoren, Verlaufshilfsmittel, Dispersionshilfsmittel, Flammschutzmittel, Optische Aufheller, Antistatika, Treibmittel, Vulkanisationsaktivatoren wie beispielsweise Zinkoxid oder Stearinsäure, Vulkanisationsbeschleuniger wie beispielsweise Mercaptobenzothiazol oder Dibenzothiazid-disulfid, Vulkanisationsmittel wie beispielsweise Schwefel oder organische Peroxide, Ladungssteuermittel.

Bevorzugte erfindungsgemässe Zusammensetzungen enthalten als weitere Additive zusätzlich eine oder mehrere Komponenten aus der Gruppe der Pigmente, Farbstoffe, Füllstoffe, Verlaufshilfsmittel, Dispersionshilfsmittel, Weichmacher, Vulkanisationsaktivatoren, Vulkanisationsbeschleuniger, Vulkanisationsmittel, Ladungssteuermittel, Haftungsverbesserer, Lichtstabilisatoren oder Antioxidantien wie beispielsweise phenolische Antioxidantien, organische Phosphite oder Phosphonite; und/oder Thiosynergisten.

Die zusätzlichen Additive werden beispielsweise in Konzentrationen von 0,01 bis 10 %, bezogen auf das Gesamtgewicht des zu stabilisierenden hellen Elastomers, zugesetzt.

Die Einarbeitung der Komponente (b) sowie gegebenenfalls weiterer Additive in das helle Elastomer erfolgt nach bekannten Methoden, beispielsweise vor oder während der Formgebung oder Vulkanisation oder auch durch Aufbringen der gelösten oder dispergierten Komponente (b) auf das helle Elastomer, gegebenenfalls unter nachträglichem Verdunsten des Lösungsmittels. Die Komponente (b) und gegebenenfalls weitere Additive können auch in Form eines Masterbatches, der diese beispielsweise in einer Konzentration von 2,5 bis 25 Gew.-% enthält, dem zu stabilisierenden hellen Elastomer zugesetzt werden.

Die Komponente (b) und gegebenenfalls weitere Additive können auch vor oder während der Polymerisation von synthetischen hellen Elastomeren oder vor der Vernetzung zugegeben werden.

Die Komponente (b) und gegebenenfalls weitere Additive können in reiner Form oder in Wachsen, Oelen oder Polymeren verkapselt in das zu stabilisierende helle Elastomer eingearbeitet werden.

Die Komponente (b) und gegebenenfalls weitere Additive können auch auf das zu stabilisierende helle Elastomer aufgesprüht werden. Sie sind in der Lage, andere Zusätze (z.B. die oben angegebenen herkömmlichen Additive) bzw. deren Schmelzen zu verdünnen, so dass sie auch zusammen mit diesen Zusätzen auf das zu stabilisierende helle Elastomer aufgesprüht werden können. Besonders vorteilhaft ist die Zugabe durch Aufsprühen während der Desaktivierung der Polymerisationskatalysatoren, wobei z.B. der zur Desaktivierung verwendete Dampf zum Versprühen verwendet werden kann.

Die so stabilisierten hellen Elastomere können in verschiedenster Form angewendet werden, z.B. als Bändchen, Formmassen, Profile, Förderbänder oder Reifen (Pneu).

Die Verbindungen der Formel I, welche neu sind, sind ebenfalls Gegenstand der vorliegenden Erfindung.

Die Erfindung betrifft daher auch auch Verbindungen der Formel I 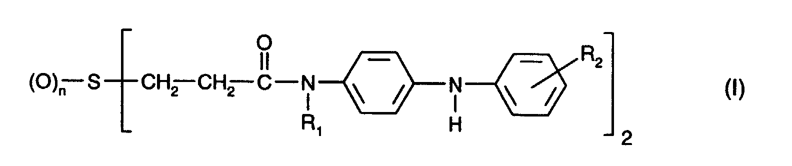 worin
R₁ Wasserstoff, C₁-C₁₂-Alkyl, Cyclohexyl, Phenyl oder Benzyl bedeutet,
R₂ Wasserstoff oder C₁-C₄-Alkyl darstellt, und
n die Zahl 0, 1 oder 2 bedeutet, mit der Bedingung, dass die Verbindung der Formel (101) 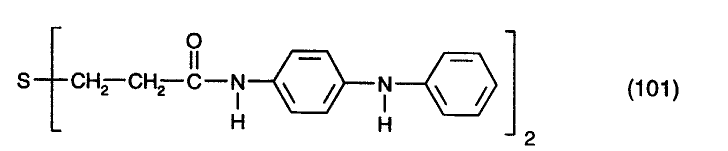 ausgeschlossen ist.

Bevorzugte Gruppen von neuen Verbindungen der Formel I entsprechen den in den oben für die erfindungsgemässen Zusammensetzungen ausgedrückten Bevorzugungen.

Besonders bevorzugt sind die neuen Verbindungen der Formel I, worin
R₁ Wasserstoff oder C₃-C₈-Alkyl bedeutet,
R₂ Wasserstoff darstellt, und
n die Zahl 0 oder 1 bedeutet, mit der Bedingung, dass die Verbindung der Formel (101) ausgeschlossen ist.

Die neuen Verbindungen der Formel I können beispielsweise ausgehend von den bekannten Verbindungen der Formel II 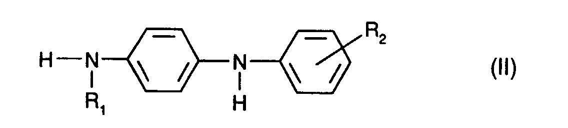 mit einem halben Equivalent Thio-3,3-bis-propionsäuredichlorid [Herstellung siehe H. Schmid et al., Helvetica Chimica Acta 34, 894-897 (1951)] zu den Verbindungen der Formel I, worin n = 0 ist, umgesetzt werden. Die Herstellung der Verbindungen der Formel I, worin n die Zahl 1 oder 2 bedeutet, erfolgt beispielsweise durch Oxidation der Verbindungen der Formel I, worin n = 0 ist, mit einer wässrigen Wasserstoffperoxid-Lösung.

Ein weiterer Gegenstand der vorliegenden Erfindung ist ein Verfahren zur Stabilisierung und zur Verminderung der Verfärbung von hellen Elastomeren, dadurch gekennzeichnet, dass man diesen mindestens eine Komponente (b) einverleibt oder auf diese aufbringt.

Eine bevorzugte Ausführungsform der vorliegenden Erfindung ist die Verwendung der Komponente (b) als farbstabile und nicht-verfärbende Stabilisatoren und Antiozonantien für helle Elastomere gegen oxidativen, thermischen, licht- oder ozoninduzierten Abbau.

Die folgenden Beispiele erläutern die Erfindung weiter. Angaben in Teilen oder Prozenten beziehen sich auf das Gewicht.

### Beispiel 1: Herstellung des Thio-3,3-bis-propionsäure-di-(4-phenylamino)anilids [Verbindung (101)]

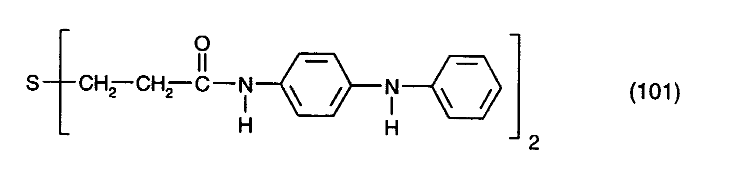

Zu einer auf 0.5°C gekühlten Lösung von 43,29 g (0,235 Mol) 4-Aminodiphenylamin und 25,3 g (0,351 Mol) Pyridin in 240 ml Dimethylformamid wird während einer Stunde 27,8 g (0,129 Mol) Thio-3,3-bis-propionsäuredichlorid [Herstellung siehe H. Schmid et al., Helvetica Chimica Acta 34, 894-897 (1951)] getropft. Anschliessend werden 120 ml Wasser zugegeben und das ausgefallene Produkt filtriert. Der Rückstand wird dreimal mit je 120 ml warmem (80°C) Wasser gewaschen und anschliessend im Trockenschrank unter Wasserstrahlvakuum bei ca. 40°C getrocknet. Kristallisation des Rückstandes aus Dimethylformamid/Toluol liefert 44,8 g (74,5 %) eines weissen Pulvers, Smp. 216,5-217°C [Verbindung (101)]. Analyse berechnet: C 70,56; H 5,92; N 10,97; S 6,28 %. Analyse gefunden: C 70,70; H 5,93; N 10,97; S 6,04 %.

### Beispiel 2: Herstellung des Thio-3,3-bis-propionsäure-di-(N-isopropyl-4-phenyiamino)anilids [Verbindung (102)]

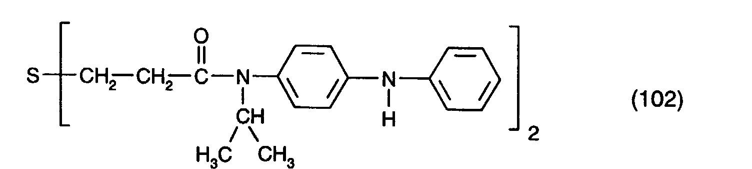

Zu einer auf -20°C gekühlten Lösung von 70,1 g (0,31 Mol) 4-lsopropylamino-diphenylamin [Vulkanox®4010, Bayer] und 33,3 (0,465 Mol) Pyridin in 370 ml Dimethylformamid wird während einer Stunde 40 g (0,186 Mol) Thio-3,3-bis-propionsäuredichlorid [Herstellung siehe H. Schmid et al., Helvetica Chimica Acta 34, 894-897 (1951)] getropft. Anschliessend werden 800 ml Toluol und 800 ml Wasser zugegeben und auf ca. 95° erwärmt. Die heisse organische Phase wird abgetrennt, mit heissem Wasser gewaschen, über Natriumsulfat getrocknet und am Vakuumrotationsverdampfer eingeengt. Kristallisation des Rückstandes aus Toluol und Trocknung der Kristalle im Hochvakuumtrockenschrank bei 125-130°C liefert 68,2 g (74 %) eines weissen Pulvers, Smp. 144,5-145,5°C [Verbindung (102)]. Analyse berechnet: C 72,69; H 7,12; N 9,42; S 5,39 %. Analyse gefunden: C 72,59; H 7,07; N 9,48; S 5,39 %.

### Beispiel 3: Herstellung des Sulfinyl-3,3-bis-propionsäure-di-(N-isopropyl-4-phenylamino)anilids [Verbindung (103)]

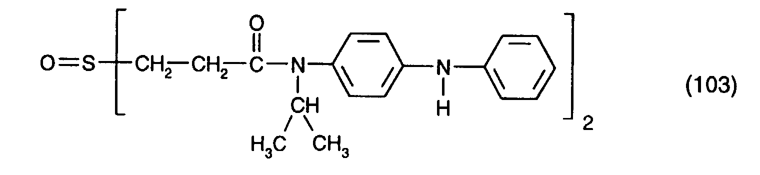

Zu einer Lösung von 22,3 g (0,037 Mol) der Verbindung (102) [Beispiel 2] in 100 ml Aceton und 200 ml Methylenchlorid wird bei Raumtemperatur 8,49 g (0,074 Mol) einer 30 % wässrigen H₂O₂-Lösung gegeben und das Reaktionsgemisch während 64 Stunden bei Raumtemperatur gerührt. Anschliessend wird das Lösungsmittel am Vakuumrotationsverdampfer eingeengt und der Rückstand in Chloroform gelöst. Die organische Phase wird mit Wasser gewaschen, über Natriumsulfat getrocknet und am Vakuumrotationsverdampfer eingeengt. Kristallisation des Rückstandes aus Methylenchlorid/Aceton liefert 19,4 g (85 %) eines weissen Pulvers, Smp. 176,0-176,5°C [Verbindung (103)]. Analyse berechnet: C 70,79; H 6,93; N 9,17; S 5,25 %. Analyse gefunden: C 69,98; H 6,93; N 9,12; S 4,96 %.

### Beispiel 4: Stabilisierung von hellem SBR-Vulkanisat.

100 Gewichstteile Califlex®S.1502 ("Styrol-Butadien-Rubber", Firma Shell) werden in einem Mischwalzwerk bei 60°C mit 30,0 Gewichtsteilen Kronos®CL 220 [Titandioxid (Pigment), Firma Kronos Titan GmbH], 30,0 Gewichtsteilen Aktisil®MM [Kaolin (Füllstoff), Firma Hoffmann Mineral Neuburg/Donau], 5,0 Gewichtsteilen Naftolen®N 401 [Weichmacher, Firma Metallgesellschaft], 10,0 Gewichtsteilen Zinkoxid [Vulkanisationsaktivator], 2,0 Gewichtsteilen Stearinsäure [Vulkanisationsaktivator], 2,0 Gewichtsteilen Schwefel [Vulkanisationsmittel], 1,0 Gewichtsteil Vulkacit®MOZ [Vulkanisationsbeschleuniger, Firma Bayer], 0,25 Gewichtsteile Vulkacit®Thluram [Vulkanisationsbeschleuniger, Firma Bayer] und 2,0 Gewichtsteile des zu prüfenden Stabilisators gemäss Tabelle 1 zu einer homogenen Mischung verarbeitet, wobei das Vulkanisationssystem (Schwefel, Vulkacit®MOZ und Vulkacit®Thiuram) erst am Ende des Mischprozesses zugesetzt wird. Die Mischung wird in elektrischen Heizpressen bei 150°C bis T95 der Rheometerkurven zu 2 mm dicken, 21 cm langen und 8,0 cm breiten Elastomerplatten vulkanisiert.

An einem Teil der so erhaltenen Elastomerplatten wird die Einwirkung von Ozon nach der Norm ASTM D 3395-86 bei dynamischer Dehnung geprüft. Dabei werden die Platten zuerst während 30 Tagen in einem Normklima [23/50 SN-ISO 291] gelagert. Anschliessend werden 20 cm mal 1 cm grosse Prüfkörper ausgestanzt und während 96 Stunden der Ozonatmosphäre ausgesetzt (Ozongehalt: 50 pphm; Temperatur: 40°C; Feuchtigkeit: 50 % rel.; Dehnung: 0 bis 25 %; Dehngeschwindigkeit: 0,5 Hz; Anzahl Lastwechsel: ca. 173'000). Die Testplatten werden dann bezüglich Rissbildung gemäss ASTM D 3395-86 beurteilt. Stufe 0 bedeutet keine Risse; Stufe 1 bedeutet schmale flache Risse; Stufe 2 bedeutet mässig breite, mässig tiefe Risse, deutlich sichtbar; Stufe 3 bedeutet breite und tiefe Risse. Je geringer die Stufenzahl, desto besser ist die Stabilisierung der Elastomerplatten. Die Resultate sind in Tabelle 1 zusammengefasst

Der andere Teil der Elastomerplatten wird während 10 Wochen bei Raumtemperatur in einer normalen Laboratmosphäre am diffusen Tageslicht gelagert. Anschliessend wird von diesen Platten die ΔL-Farbe nach DIN 6167, die einer Skala von 0 bis 100 entspricht, bestimmt. Keine Verfärbung bedeutet einen Wert von 100. Die Resultate sind in Tabelle 1 zusammengefasst.

**Tabelle 1:**

| Beispiele | Stabilisator | Rissbildung nach ASTM D 3395-86 | ΔL-Farbe nach DIN 6167 |
|---|---|---|---|
| Beispiel 4a^{a)} | - | Stufe 1-2 | 96 |
| Beispiel 4b^{a)} | 2,0 phr^{c)} Vulkanox®4010^{d)} | Stufe 0 | 37 |
| Beispiel 4c^{b)} | 2,0 phr^{c)} Verbindung (101) | Stufe 1 | 94 |
| Beispiel 4d^{b)} | 2,0 phr^{c)} Verbindung (103) | Stufe 0-1 | 75 |

a) Vergleichsbeispiele.
b) erfindungsgemässe Beispiele.
c) phr bedeutet "**p**arts per **h**undred of **r**ubber" (Teile pro hundert Teile Gummi)
d) Vulkanox®4010 (Bayer) bedeutet 4-lsopropylamino-diphenylamin der Formel A

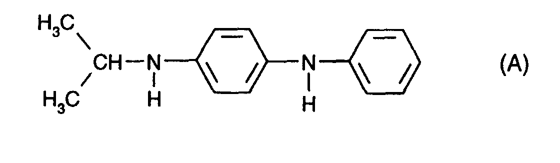

## Patentansprüche

1. Zusammensetzung enthaltend
a) ein dem oxidativen, thermischen, licht- oder ozoninduzierten Abbau unterworfenes helles Elastomer, und
b) mindestens eine Verbindung der Formel I 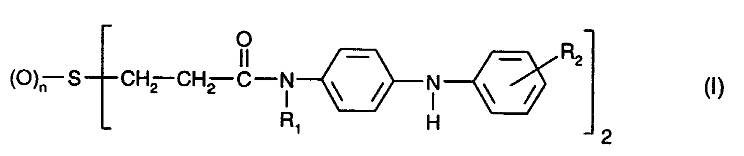 worin
R₁ Wasserstoff, C₁-C₁₂-Alkyl, Cyclohexyl, Phenyl oder Benzyl bedeutet,
R₂ Wasserstoff oder C₁-C₄-Alkyl darstellt, und
n die Zahl 0, 1 oder 2 bedeutet.

2. Zusammensetzung gemäss Anspruch 1, enthaltend als Komponente (b) mindestens eine Verbindung der Formel I, worin
R₁ Wasserstoff oder C₃-C₈-Alkyl bedeutet,
R₂ Wasserstoff oder Methyl darstellt, und
n die Zahl 0 oder 1 bedeutet.

3. Zusammensetzung gemäss Anspruch 1, enthaltend als Komponente (b) mindestens eine Verbindung der Formel I, worin
R₁ Wasserstoff oder C₃-C₈-Alkyl bedeutet,
R₂ Wasserstoff darstellt, und
n die Zahl 0 oder 1 bedeutet.

4. Zusammensetzung gemäss Anspruch 1, worin die Komponente (a) ein helles Polydien-Vulkanisat oder eine helles halogenhaltiges Polydien-Vulkanisat ist.

5. Zusammensetzung gemäss Anspruch 1, worin die Komponente (a) ein helles Styrol-Butadien-Copolymer-Vulkanisat ist.

6. Zusammensetzung gemäss Anspruch 1, enthaltend neben den Komponenten (a) und (b) zusätzlich weitere Additive.

7. Zusammensetzung gemäss Anspruch 6, enthaltend als weitere Additive zusätzlich eine oder mehrere Komponenten aus der Gruppe der Pigmente, Farbstoffe, Füllstoffe, Verlaufshilfsmittel, Dispersionshilfsmittel, Weichmacher, Vulkanisationsaktivatoren, Vulkanisationsbeschleuniger, Vulkanisationsmittel, Ladungssteuermittel, Haftungsverbesserer, Antioxidantien oder Lichtstabilisatoren.

8. Zusammensetzung gemäss Anspruch 6, enthaltend als weitere Additive phenolische Antioxidantien, organische Phosphite oder Phosphonite; und/oder Thiosynergisten.

9. Zusammensetzung gemäss Anspruch 1, worin die Komponente (b) in einer Menge von 0,2 bis 10 % bezogen auf das Gewicht der Komponente (a) vorliegt.

10. Neue Verbindungen der Formel I 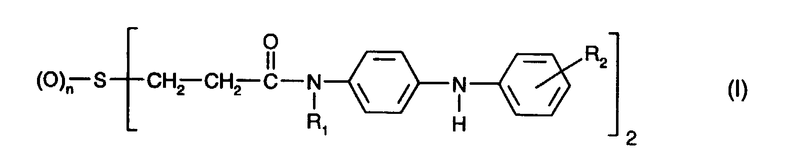 worin
R₁ Wasserstoff, C₁-C₁₂-Alkyl, Cyclohexyl, Phenyl oder Benzyl bedeutet,
R₂ Wasserstoff oder C₁-C₄-Alkyl darstellt, und
n die Zahl 0, 1 oder 2 bedeutet, mit der Bedingung, dass die Verbindung der Formel (101) 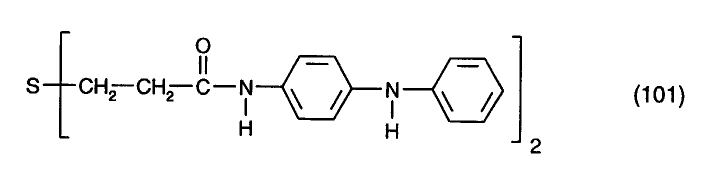 ausgeschlossen ist.

11. Verbindung gemäss Anspruch 10, worin
R₁ Wasserstoff oder C₃-C₈-Alkyl bedeutet,
R₂ Wasserstoff darstellt, und
n die Zahl 0 oder 1 bedeutet.

12. Verfahren zur Stabilisierung und zur Verminderung der Verfärbung von hellen Elastomeren, dadurch gekennzeichnet, dass man diesen mindestens eine Komponente (b) gemäss Anspruch 1 einverleibt oder auf diese aufbringt.

13. Verwendung der Komponente (b) gemäss Anspruch 1 als farbstabile und nicht-verfärbende Stabilisatoren und Antiozonantien für helle Elastomere gegen oxidativen, thermischen, licht- oder ozoninduzierten Abbau.
